Europäisches Patentamt

European Patent Office

Office européen des brevets

(11) Publication number: **0 429 967 B1**

# EUROPEAN PATENT SPECIFICATION

(45) Date of publication of patent specification: **05.10.94**　(51) Int. Cl.5: **C07C 21/09**, C07C 17/02

(21) Application number: **90121834.7**

(22) Date of filing: **15.11.90**

(54) **Process for the liquid phase chlorination of 1,3-butadiene.**

(30) Priority: **17.11.89 US 438549**

(43) Date of publication of application:
**05.06.91 Bulletin 91/23**

(45) Publication of the grant of the patent:
**05.10.94 Bulletin 94/40**

(84) Designated Contracting States:
**DE FR GB**

(56) References cited:
**FR-A- 2 147 026
GB-A- 1 435 826
US-A- 2 369 117
US-A- 2 948 760**

(73) Proprietor: **E.I. DU PONT DE NEMOURS AND COMPANY
1007 Market Street
Wilmington Delaware 19898 (US)**

(72) Inventor: **Harris, Alexander Thomas
1605 Princeton Avenue
Metairie, Louisiana 70003 (US)**

(74) Representative: **von Kreisler, Alek,
Dipl.-Chem. et al
Patentanwälte
von Kreisler-Selting-Werner
Postfach 10 22 41
D-50462 Köln (DE)**

## Description

This invention relates to an improved process for the liquid phase chlorination of 1,3-butadiene to 3,4-dichlorobutene-1 and trans-1,4-dichlorobutene-2, which is characterized by high yield, excellent specificity, low operating temperature, and results in ready and economical removal of unreacted 1,3-butadiene and solvent for reuse in the system.

1,4-Dichlorobutene-2 is a valuable intermediate in the manufacture of certain important polyamides, such as Nylon 66, while 3,4-dichlorobutene-1 is an equally valuable intermediate in the manufacture of chloroprene, which is the basic monomer in the manufacture of a class of important synthetic rubbers known as the Neoprenes.

It is known to chlorinate 1,3-butadiene in the presence of halogenated solvents in the liquid phase, for example in systems catalyzed by soluble quaternary ammonium, pyridinium, phosphonium, and sulfonium chlorides, as described in British Patent 1,435,826, or in the presence of ferric chloride catalysts, as described in German Patent 2357194 to produce 3,4-dichlorobutene-1 and 1,4-dichlorobutene-2. Vapor phase processes are also known and have been used on an industrial scale for many years. Vapor phase processes require large excesses of 1,3-butadiene that require recycling. The reaction is characterized by low yield, non-specifity and high temperatures of the order of 225-300°C. Despite the fact that liquid phase chlorination results in higher yields of the desired 3,4-dichlorobutene-1 and trans-1,4-dichlorobutene-2 products compared to vapor phase processes, liquid phase chlorination has not been commercially attractive. This is primarily due to the operating costs and large investment required for equipment to remove solvent from the low boilers, dichlorobutene products, and high boilers in several distillation columns.

An object of this invention is to provide a liquid phase chlorination process that simplifies recovery of the valuable dichlorobutene products and unreacted 1,3-butadiene, especially a process that precludes the necessity for secondary distillation facilities to remove large amounts of solvent from the dichlorobutenes. Also, the process of this invention reduces the amount of by-products formed during the chlorination of 1,3-butadiene with a concomitant increase in the yield of the desired dichlorobutenes compared to both other liquid phase processes and vapor phase processes.

The present process provides for a low temperature liquid phase chlorination reaction of 1,3-butadiene in which solvent is readily removed from the chlorinated products. More specifically the process is directed to the liquid phase chlorination of 1,3-butadiene to produce a mixture of 3,4-dichlorobutene-1 and trans-1,4-dichlorobutene-2 which comprises contacting in an evaporatively cooled reactor 1,3-butadiene with elemental chlorine in a solvent in substantially oxygen-free atmosphere in the presence of a chlorination catalyst at a temperature of 25-100°C and at a pressure sufficient to give the resulting reaction mixture a boiling point of from 25-100°C, said solvent being the hydrocarbons butane or pentane or a fluorinated solvent represented by the formula:

$$CR_3(CR'_2)_m(CR''_2)_nR$$

where R is independently hydrogen, fluorine, chlorine or bromine, R' is hydrogen, R'' is independently fluorine, chlorine or bromine, m and n are 0-3, with the proviso that terminal carbon atoms are independently perhalogenated or fully hydrogenated, said solvents having boiling points of from -15°C to 40°C at atmospheric pressure, the solvent to dichlorobutene ratio is from 2.5:1 to 10:1 and said solvent being substantially inert to reaction with elemental chlorine at reaction conditions, separating the unreacted 1,3-butadiene and solvent from the dichlorobutenes by condensing the overhead vapor of solvent and unreacted 1,3-butadiene and recycling it as a single stream to the reactor for reuse and recovering the dichlorobutenes.

The process of the present invention involves feeding elemental chlorine and 1,3-butadiene to an evaporatively cooled reactor containing a particular solvent. Minor amounts of a free radical inhibitor (excluding free oxygen or air) are added to reduce or eliminate free radical reactions and the formation of excessive amounts of hydrogen chloride. A catalyst, or catalyst precursor which forms the chlorination catalyst in situ, is added to increase the rate of formation of the dichlorobutenes and yields thereof. The reactor is operated at temperatures of from 25-100°C. The 1,3-butadiene and elemental chlorine react in the liquid phase to form dichlorobutene. The heat of reaction is removed by vaporization of some of the solvent and unreacted 1,3-butadiene. The vapors of the unreacted 1,3-butadiene and solvent that evaporate are condensed in a reflux condenser and returned to the reactor. The bottoms from the reactor which is the product stream containing dichlorobutene, unreacted 1,3-butadiene and solvent, is fed to a stripper column or flasher where the crude dichlorobutene is separated by stripping the low boiling solvent and 1,3-

EP 0 429 967 B1

butadiene (about 2 wt. %) in the overhead. The crude dichlorobutene containing minor amounts of low and high boiling by-products is fed to refining columns for purification. The overhead vapor of solvent and unreacted 1,3-butadiene from the stripping column is condensed and returned to the reactor for reuse.

The reaction of elemental chlorine, e.g., gaseous chlorine, with 1,3-butadiene is highly exothermic. The solvent used in the process acts as a diluent to improve yield and to evaporatively cool the reactor. As long as there is an excess of solvent and/or 1,3-butadiene, the reactor contents will boil and remove the heat of reaction.

The reaction is conducted substantially in the absence of oxygen. The presence of oxygen, e.g. air, will result in the formation of liquid butadiene peroxides that are unstable and, therefore, air must be substantially excluded.

An important condition for obtaining the results sought requires the use of particular solvents in the process. The solvents used in the process are the hydrocarbons butane or pentane or a fluorinated solvent represented by the formula:

$$CR_3(CR'_2)_m(CR''_2)_nR$$

where R is independently hydrogen, fluorine, chlorine or bromine, R′ is hydrogen, R″ is independently fluorine, chlorine or bromine, m and n are 0-3, with the proviso that the terminal carbon atoms are independently perhalogenated or fully hydrogenated, further, the solvents have boiling points of -15°C to 40°C at atmospheric pressure, the solvent to dichlorobutene product ratio is from 2.5:1 to 10:1, and said solvents are substantially inert to reaction with elemental chlorine at reaction conditions.

The solvents used in the chlorination process have boiling points relatively close to the boiling point of 1,3-butadiene (-4°C). i.e., 40°C to -15°C. The selection of such solvent allows both the solvent and unreacted 1,3-butadiene to be separated from the dichlorobutene by stripping in one step. The fluorinated solvent can be represented by the formula:

$$CR_3(CR'_2)_m(CR''_2)_nR$$

with the values for R, R′, R″ and m and n as given above. Preferably in the above formula R and R″ are fluorine, chlorine or bromine and m equals 0. Perfluorinated solvents wherein R and R″ are fluorine and m equals 0 are especially effective but are subject to environmental restrictions. If the boiling point of the solvent is above 40°C, the solvent will be difficult to separate from the low boiling by-products and the product dichlorobutenes without high investments in distillation equipment. On the other hand, if the boiling point of the solvent is too low, i.e., below -15°C, it becomes difficult to condense, and low temperature refrigeration and high pressure compression are required to avoid excessive vent losses. Solvents that are used in the process of this invention are the hydrocarbons butane and pentane and representative fluorinated solvents include dichlorodifluoromethane, 1,1-dichlorotetrafluoroethane, 1,2-dichlorotetrafluoroethane, 1-chloro-1,1-fluoroethane, 1-chloroheptafluoropropane, 1,1,1,2,2-pentafluoropropane, perfluorobutane, 2,3-di-chlorooctafluorobutane, and 2,2,3,3-tetrafluorobutane. Preferred solvents include butane, pentane, 1,1-dichloro-1-fluoroethane, and 1,2-dichlorotetrafluoroethane; the latter is especially preferred because its boiling point is very close to that of the butadiene and it is available in commercial quantities. The weight ratio of solvent to crude dichlorobutene in the reactor is from 2.5:1 to 10:1, preferably at least 6. Lower ratios lead to decreases in yield at the shorter residence times. For example, a weight ratio of 8 is quite satisfactory for residence times of about 10 minutes.

The chlorination process is conducted at a temperature of 25-100°C, preferably 40-60°C, and at a pressure sufficient to give the solution in the reactor which consists of 1,3-butadiene, dichlorobutenes and solvent, i.e., reaction mixture, a boiling point of from 25-100°C, preferably 40-60°C. The pressures under which the reaction is conducted will vary with the boiling point of the particular solvent used. In any event, the pressure employed is that sufficient to give the reaction mixture of 1,3-butadiene, the dichlorobutenes and solvent a boiling point of from 25-100°C. The heat of the reaction is removed by boiling and vaporization of solvent and 1,3-butadiene which are condensed and returned to the reactor. Residence times in the reactor in the continuous process generally range from 1.5 to 10 minutes. Residence times less than 1.5 minutes lead to a decrease in yield of dichlorobutenes and residence times in excess of 10 minutes are not economically attractive on a commercial scale.

A catalyst is added to the reaction mixture to promote the ionic chlorination reaction. Such catalysts are well known in this technology. Suitable catalysts are chloride ion sources which may be added to the reaction mixture in the form of chloride salts or in the form of materials which will react with a component of the reaction mixture to produce a chloride salt in situ, i.e., catalyst precursor. Representative examples of

3

suitable compounds which act as catalysts for the reaction are quaternary ammonium chlorides, quaternary phosphonium chlorides, and ternary sulfonium chlorides. Hydrochlorides of primary, secondary, or tertiary amines can also be used. Examples of materials which may be added to from the catalyst in situ include amines, either primary, secondary, or tertiary, or the analogous phosphides or sulfides. These compounds are capable of reacting with one or more of the chlorine-substituted materials in the reaction mixture or with hydrogen chloride to form a chloride ion source. Other examples of precursors for chloride ions are salts in which the anion is not a chloride ion but which can undergo an ion exchange reaction in the reaction medium to produce a chloride ion. Quaternary ammonium chlorides are a preferred catalyst type because they are widely available commercially as surface active agents. Representative quaternary ammonium compounds include butyltriethylammonium chloride, dilauryldimethylammonium chloride, amyltriethylammonium chloride, tetraoctylammonium chloride, hexyltrimethylammonium chloride, and the like. Suitable quaternary phosphonium compounds include, for example, tetrabutylphosphonium chloride, methyltrioctylphosphonium chloride, trimethyloctadecenylphosphonium chloride, and triethyl(2-bromoethyl)phosphonium chloride. Sulfonium compounds which may be used as catalysts include trimethylsulfonium chloride, dihexylethylsulfonium chloride, dihexylethylsulfonium chloride, methyldioctadecylsulfonium chloride, propyldibutylsulfonium chloride, arid dimethylcyclohexylsulfonium chloride. It is usually more convenient to form the catalyst in situ, for example by adding an amine as a free base which can then react to form the chloride ion source in the reaction mixture. Pyridine is particularly useful as a catalyst precursor. Other compounds which will form catalysts in situ in the reaction medium are the carboxylic acid amides such as formamide, acetamide, 2-pyrrolidone, 2-piperidone, and N-butylacetamide. The catalyst precursor concentration generally ranges from 20-200 ppm based on the amount of solvent present. Below 20 ppm there is a decrease in yield, and concentrations above 200 ppm are not cost effective.

The process is preferably, although not necessarily, carried out in the presence of free radical inhibitors. Conventional free radical inhibitors include phenols such as 4-tert-butyl catechol, aromatic amines, such as phenyl alpha-naphthylamine, phenothiazine, and N-nitrosodiphenylamine, and other inhibitors, such as sulfur. Oxygen should not be used as a free radical inhibitor in the process of this invention because of the formation of unstable peroxide compounds with butadiene. Practical inhibitor concentrations have been found to be about 20-80 ppm based on the amount of solvent present. Concentrations below 20 ppm lead to decreases in yield, while concentrations over 80 ppm are not economical.

The reaction mixture or effluent coming from the chlorination reactor is fed to a refining column for separating the unreacted 1,3-butadiene and solvent from 3,4-dichlorobutene-1 and trans-1,4-dichlorobutene-2. The physical separation of the reaction stream into the two principal streams, i.e., 1,3-butadiene/solvent and the dichlorobutenes, can be accomplished by suitable means well known in the art. The physical separation of the reaction mixture can be performed, for example, in a stripper in which the reaction mixture is fed into the stripper column. Heat supplied to the bottom of the column causes vaporization of the reaction components. The lower boiling components consisting of 1,3-butadiene and solvent go overhead for recycling, usually as a single stream, to the reactor and the higher boiling components containing the dichlorobutenes are removed from the bottom of a column for further purification. Also conventional single stage flashers can be used to separate the reaction mixture for recirculation of the 1,3-butadiene/solvent to the reactor and recovery of the dichlorobutenes. Conventional distillation columns can also be used. Separation of the streams using a stripper column is preferred.

The following examples illustrate the invention wherein parts and percentages are by weight unless otherwise indicated.

Example 1

A cylindrical evaporatively cooled nickel pressure reactor equipped with an agitation means and having interior dimensions of 23 mm in height by 7.8 mm in diameter was continuously fed with a stream of gaseous chlorine and a second stream consisting of 1,3-butadiene in the solvent 1,2-dichlorotetrafluoroethane. The fluorinated solvent has a boiling point of 4°C at atmospheric pressures. The chlorine was introduced at the rate of 0.17 g/s. The rate of addition of the butadiene/solvent stream was 3.2 g/s (0.21g butadiene/s and 3g 1,2-dichlorotetrafluoroethane/s). The butadiene had a purity of 99.3%. The reactor was operated at a 15 cm liquid level which gave a residence time of 3.2 minutes at the feed rates used. An inhibitor, phenyl alpha-naphthylamine, dissolved in pyridine (chlorination catalyst precursor), was added to the reactor to give concentrations of 50 and 100 ppm respectively based on the solvent 1,2-dichlorotetrafluoroethane. The ratio of solvent to dichlorobutenes was 10. The reactor was operated at 323K (50°C) and a pressure of 445kPa. The reaction mixture, containing 3,4-dichlorobutene-1 and trans-1,4-dichlorobutene-2, unreacted 1,3-butadiene and solvent was fed to a stripper column. Unreacted 1,3-

EP 0 429 967 B1

butadiene and solvent were vaporized and removed overhead in the stripper column, subsequently condensed, and recycled to the reactor for reuse in the system. The dichlorobutenes were removed from the bottom of the column. The yield of dichlorobutenes was 96.3%, as determined by gas chromatography using a J&W fused silica capillary (Alltech 9385) DB-5 "Durabond" 60M x 0.25 mm I.D. column with one coat of bonded 95% dimethyl, 5% diphenyl silicone operated at a maximum temperature of 250°C. The product had the following composition by weight:

| | |
|---|---|
| 3,4-dichlorobutene-1 | 46% |
| cis-1,4-dichlorobutene-2 | 1% |
| trans-1,4-dichlorobutene-2 | 47% |
| trichlorobutenes | 0.9% |
| tetrachlorobutanes | 4.2% |
| monochlorobutadienes | 0.6% |
| other | 0.5% |

Example 2

The procedure described in Example 1 was repeated except n-pentane was used as the solvent. Gaseous chlorine and a second stream consisting of 1,3-butadiene in the solvent n-pentane of 99.3% purity were fed to the reactor. The solvent has a boiling point of 36°C. The chlorine was introduced at the rate of 0.22 g/s. The rate of addition of the butadiene/solvent stream was 1.5 g/s (0.27 butadiene/second) and 1.2 g n-pentane/s). The butadiene had a purity of 99.8%. The reactor was operated at a 14 cm liquid level which gave a residence time of 3 minutes at the feed rates used. An inhibitor, phenyl alpha-naphthylamine, dissolved in pyridine (chlorination catalyst precursor), was added to the reactor to give concentrations of 100 and 200 ppm respectively based on the pentane solvent. The ratio of solvent to dichlorobutenes was 3. The reactor was operated at 330 K (57°C) and a pressure of 310 kpa. The reaction mixture containing the dichlorobutenes, unreacted 1,3-butadiene and solvent was fed to a stripper column. Unreacted 1,3-butadiene and solvent were vaporized and removed overhead in the stripper column, subsequently condensed, and recycled to the reactor for reuse in the system. The dichlorobutenes were removed from the bottom of the column. The yield of dichlorobutenes was 96.7% as determined by gas chromatography using a J&W fused silica capillary (Alltech 9385) DB-5 "Durabond" 60M x 0.25 mm I.D. column with one coat of bonded 95% dimethyl, 5% diphenyl silicone operated at a maximum temperature of 250°C. The product had the following composition by weight:

| | |
|---|---|
| monochlorobutadienes | 1.0% |
| 3,4-dichlorobutene-1 | 48% |
| cis-1,4-dichlorobutene-2 | 1.3% |
| trans-1,4-dichlorobutene-2 | 45% |
| trichlorobutenes | 0.8% |
| tetrachlorobutanes | 3.5% |
| other | 0.4% |

Example 3

An evaporatively cooled reactor having a diameter of 5 cm and a length of 15 cm was equipped with a magnetic mixer and an 0.1 sq. meter reflux condenser. Gaseous chlorine, liquid 1,3-butadiene, and the solvent n-butane, having a purity of 99.7%, was fed to the reactor. The solvent has a boiling point of -4°C. The chlorine was introduced at the rate of 0.1 g/s. The rate of addition of the butadiene was 0.12 g/s. The rate of addition of the solvent containing the catalyst precursor (pyridine) and inhibitor (phenyl alpha-naphthylamine) was 0.46 g/s. The butadiene had a purity of 99.3%. The reactor was operated at a 7.6 cm liquid level which gave a residence time of 2.5 minutes at the feed rates used. The inhibitor, phenyl alpha-naphthylamine, dissolved in pyridine (chlorination catalyst precursor), was added to the solvent to give concentrations of 40 and 170 ppm respectively based on the solvent n-butane. The ratio of solvent to dichlorobutenes was 2.5. The reactor was operated at 320 K (48°C) and a pressure of 448 kpa. The

5

reaction mixture containing 3,4-dichlorobutene-1 and trans-1,4-dichlorobutene-2, unreacted 1,3-butadiene and solvent was fed to a stripper column. Unreacted 1,3-butadiene and solvent were vaporized and removed overhead in the stripper column, subsequently condensed, and recycled to the reactor for reuse in the system. The dichlorobutenes were removed from the bottom of the column. The yield of dichlorobutenes was 98% as determined by gas chromatography using J&W fused silica capillary (Alltech 9385) DB-5 "Durabond" 60M x 0.25 mm I.D. column with one coat of bonded 95% dimethyl, 5% diphenyl silicone operated at a maximum temperature of 250°C. The product had the following composition by weight:

| monochlorobutadienes | 1.8% |
|---|---|
| 3,4-dichlorobutene-1 | 54% |
| cis-1,4-dichlorobutene-2 | 0.7% |
| trans-1,4-dichlorobutene-2 | 43% |
| trichlorobutenes | Trace |
| tetrachlorobutanes | Trace |
| other | Trace |

Example 4

The procedure described in Example 3 was repeated except that the stream of gaseous chlorine was introduced at a rate of 3.6 g/minute and 1,3-butadiene was fed to the reactor at a rate of 4.1 g/minute. The solvent used was 1,1-dichloro-1-fluoroethane having a boiling point of 32°C, and introduced at the rate of 64 g/minute. The reactor was operated at 7.6 cm liquid level which gave a residence time of 2.5 minutes at the feed rates used. An inhibitor, phenyl alpha-naphthylamine, dissolved in pyridine (catalyst precursor) was added to the reactor to give concentrations of 40 and 100 ppm respectively based on the solvent 1,1-dichloro-1-fluoroethane. The ratio of solvent to the dichlorobutenes was 10. The reactor was operated at 318K (45°C) and a pressure of 172 kPa. The reaction mixture containing the dichlorobutenes, unreacted 1,3-butadiene and solvent was fed to a stripper column. Unreacted 1,3-butadiene and solvent were vaporized and removed overhead in the stripper column, subsequently condensed, and recycled to the reactor for reuse in the system. The dichlorobutenes were removed from the bottom of the column. The yield of dichlorobutenes was 96.3% as determined by gas chromotography using a J&W fused silica capillary 60M x 0.25 mm I.D. column operated at maximum temperature of 250°C. The product had the following composition by weight:

| 3,4-dichlorobutene-1 | 45.37% |
|---|---|
| cis-1,4-dichlorobutene-2 | 0.99% |
| trans-1,4-dichlorobutene-2 | 47.19% |
| trichlorobutenes | 0.75% |
| tetrachlorobutane | 3.73% |
| monochlorobutadiene | 1.2% |
| other | 0.76% |

Examples 5-15

The procedure described above in Example 1 was repeated except that the parameters given in Table 1 below were substituted for those used in Example 1.

TABLE I

| EXAMPLE | YIELD* (%) | BUTADIENE/Cl₂ FEED MOL RATIO | SOLVENT** PRODUCT RATIO | REACTION TEMP (°C) | RESIDENCE TIME (MIN) | PYRIDINE (ppm) IN SOLVENT*** | INHIBITOR (ppm) IN SOLVENT**** |
|---|---|---|---|---|---|---|---|
| 5 | 97 | 1.5 | 10 | 47 | 1.5 | 60 | 30 |
| 6 | 93 | 1.1 | 7.8 | 40 | 3.1 | 96 | 48 |
| 7 | 95.6 | 1.85 | 8.2 | 40 | 3.5 | 99 | 49 |
| 8 | 96.2 | 1.6 | 6 | 40 | 3.3 | 103 | 52 |
| 9 | 96.3 | 1.7 | 10.7 | 40 | 3.2 | 100 | 50 |
| 10 | 95.6 | 1.85 | 8.2 | 30 | 3.4 | 99 | 49 |
| 11 | 96.3 | 1.78 | 10 | 60 | 3.3 | 97 | 48 |
| 12 | 96.3 | 1.67 | 10 | 52 | 2.1 | 103 | 51 |
| 13 | 95.8 | 1.74 | 10.6 | 52 | 10.3 | 95 | 47 |
| 14 | 95.2 | 1.84 | 9.9 | 40 | 2.1 | 20 | 48 |
| 15 | 95.2 | 1.89 | 10.1 | 40 | 2.1 | 50 | 49 |

* yield % of 3,4-dichlorobutene-1 and cis and trans 1,4-dichlorobutene-2 from 1,3-butadiene.
** weight of dichlorobutenes to solvent 1,2-dichlorotetrafluoroethane exising reactor, by wt.
*** catalyst precursor, by wt.
**** phenyl alpha-naphthylamine, by wt.

Claims

1. A process for the liquid phase chlorination of 1,3-butadiene to produce a mixture of 3,4-dichlorobutene-1 and trans-1,4-dichlorobutene-2 which comprises contacting in an evaporatively cooled reactor 1,3-butadiene with elemental chlorine in a solvent in a substantially oxygen-free atmosphere and in the presence of a chlorination catalyst at a temperature of 25-100°C and at a pressure sufficient to give the resulting reaction mixture a boiling point of from 25-100°C, said solvent being the hydrocarbons butane or pentane or a fluorinated solvent represented by the formula:

$$CR_3(CR'_2)_m(CR''_2)_nR$$

where R is independently hydrogen, fluorine, chlorine or bromine, R' is hydrogen, R'' is independently fluorine, chlorine or bromine, m and n are 0-3, with the proviso that terminal carbon atoms are independently perhalogenated or fully hydrogenated, further the solvents having boiling points of from -15°C to 40°C at atmospheric pressure, the solvent to dichlorobutene ratio is from 2.5:1 to 10:1 and said solvent being substantially inert to reaction with elemental chlorine at reaction conditions, separating the unreacted 1,3-butadiene and solvent from the dichlorobutenes by condensing the overhead vapor of solvent and unreacted 1,3-butadiene and recycling it as a single stream to the reactor and recovering the dichlorobutenes.

2. A process of Claim 1 wherein R and R'' are fluorine, chlorine or bromine and m equals 0.

3. A process of Claim 2 wherein R and R'' are fluorine.

4. A process of Claim 1 wherein R'' is fluorine.

5. A process of Claim 1 wherein the solvent in n-butane.

6. A process of Claim 1 wherein the solvent is pentane.

7. A process of Claim 1 wherein the solvent is 1,2-dichlorotetrafluoroethane.

8. A process of Claim 1 wherein the chlorination reaction is conducted in the presence of a free radical inhibitor.

9. A process of Claim 1 wherein the reaction temperature is 40-60°C.

10. A process of Claim 1 wherein elemental chlorine is gaseous chlorine.

**Patentansprüche**

1. Verfahren zur Chlorierung von 1,3-Butadien in flüssiger Phase unter Erzeugung eines Gemischs aus 3,4-Dichlorbuten-1 und trans-1,4-Dichlorbuten-2, umfassend
   das In-Kontakt-Bringen von 1,3-Butadien mit elementarem Chlor in einem durch Verdunstung gekühlten Reaktor in einem Lösungsmittel in einer im wesentlichen sauerstofffreien Atmosphäre und in Gegenwart eines Chlorierungskatalysators bei einer Temperatur von 25-100°C und bei einem Druck, der ausreicht, um dem resultierenden Reaktionsgemisch einen Siedepunkt zwischen 25 und 100°C zu verleihen, wobei es sich bei dem Lösungsmittel um die Kohlenwasserstoffe Butan oder Pentan oder um ein fluoriertes Lösungsmittel handelt, das durch die Formel:

$$CR_3(CR'_2)_m(CR''_2)_nR$$

wiedergegeben wird, worin R unabhängig Wasserstoff, Fluor, Chlor oder Brom ist, R' Wasserstoff ist, R'' unabhängig Fluor, Chlor oder Brom ist, m und n 0-3 betragen, mit der Maßgabe, daß terminale Kohlenstoffatome unabhängig perhalogeniert oder vollständig hydrogeniert sind, daß ferner die Lösungsmittel Siedepunkte zwischen -15°C bis 40°C bei Atmosphärendruck besitzen, daß das Verhältnis Lösungsmittel zu Dichlorbuten zwischen 2.5:1 und 10:1 liegt und daß das Lösungsmittel unter den Reaktionsbedingungen im wesentlichen inert gegenüber einer Reaktion mit elementarem Chlor ist;
   das Abtrennen des unreagierten 1,3-Butadiens und des Lösungsmittels von den Dichlorbutenen durch Kondensieren des Überkopfdampfes aus Lösungsmittel und unreagiertem 1,3-Butadien und dessen Zurückführen im Kreislauf als einzelner Strom zum Reaktor
   sowie die Gewinnung der Dichlorbutene.

**2.** Verfahren gemäß Anspruch 1, worin R und R" Fluor, Chlor oder Brom sind und m gleich 0 ist.

**3.** Verfahren gemäß Anspruch 2, worin R und R" Fluor sind.

**4.** Verfahren gemäß Anspruch 1, worin R" Fluor ist.

**5.** Verfahren gemäß Anspruch 1, worin das Lösungsmittel n-Butan ist.

**6.** Verfahren gemäß Anspruch 1, worin das Lösungsmittel Pentan ist.

**7.** Verfahren gemäß Anspruch 1, worin das Lösungsmittel 1,2-Dichlortetrafluorethan ist.

**8.** Verfahren gemaß Anspruch 1, worin die Chlorierungsreaktion in Gegenwart eines Inhibitors für freie Radikale durchgeführt wird.

**9.** Verfahren gemäß Anspruch 1, worin die Reaktionstemperatur 40-60°C beträgt.

**10.** Verfahren gemäß Anspruch 1, worin elementares Chlor gasförmiges Chlor ist.

## Revendications

**1.** Un procédé pour la chloration en phase liquide du butadiène-1,3 pour produire un mélange de 3,4-dichlorobutène-1 et de *trans*-1,4-dichlorobutène-2, qui consiste à mettre en contact, dans un réacteur refroidi par évaporation, du butadiène-1,3 avec du chlore élémentaire dans un solvant dans une atmosphère sensiblement exempte d'oxygène et en présence d'un catalyseur de chloration à une température de 25 à 100°C et sous une pression suffisante pour conférer au mélange réactionnel résultant un point d'ébullition de 25 à 100°C, ledit solvant étant le butane ou le pentane comme hydrocarbures ou un solvant fluoré représenté par la formule :

$$CR_3(CR'_2)_m(CR''_2)_nR$$

où R est indépendamment l'hydrogène, le fluor, le chlore ou le brome, R' est l'hydrogène, R" est indépendamment le fluor, le chlore ou le brome, m et n sont de 0 à 3, avec la condition que les atomes de carbone terminaux soient indépendamment perhalogénés ou complètement hydrogénés, les solvants ayant en outre des points d'ébullition de -15°C à 40°C à la pression atmosphérique, le rapport du solvant aux dichlorobutènes étant de 2,5:1 à 10:1 et ledit solvant étant sensiblement inerte quant à une réaction avec le chlore élémentaire dans les conditions réactionnelles, à séparer le butadiène-1,3 n'ayant pas réagi et le solvant des dichlorobutènes par condensation de la vapeur de tête constituée de solvant et de butadiène-1,3 n'ayant pas réagi et les recycler dans un même courant vers le réacteur, et à retirer les dichlorobutènes.

**2.** Un procédé de la revendication 1, dans lequel R et R" sont du fluor, du chlore ou du brome et m égale 0.

**3.** Un procédé de la revendication 2, dans lequel R et R" sont du fluor.

**4.** Un procédé de la revendication 1, dans lequel R" est le fluor.

**5.** Un procédé de la revendication 1, dans lequel le solvant est le *n*-butane.

**6.** Un procédé de la revendication 1, dans lequel le solvant est le pentane.

**7.** Un procédé de la revendication 1, dans lequel le solvant est le 1,2-dichlorotétrafluoréthane.

**8.** Un procédé de la revendication 1, dans lequel la réaction de chloration est conduite en présence d'un inhibiteur de radicaux libres.

**9.** Un procédé de la revendication 1, dans lequel la température de réaction est de 40 à 60°C.

**10.** Un procédé de la revendication 1, dans lequel le chlore élémentaire est du chlore gazeux.